# EUROPEAN PATENT APPLICATION

(11) **EP 4 243 001 A1**
(43) Date of publication of application: **13.09.2023**
(21) Application number: 21889701.5
(22) Date of filing: 26.07.2021
(51) Int. Cl.: G09B 23/28, G06T 15/00, G09B 9/00, G02B 27/01

(54) **SIMULATOR SYSTEM FOR THE SAFE TRAINING OF MEDICAL PERSONNEL**

(30) Priority: 03.11.2020 RU 2020135486
(71) Applicant: Obshchestvo S Ogranichennoj Otvetstvennost'Yu "Viarsim", Sankt-Peterburg, 197022 (RU)
(72) Inventor: KOSTYUSHOV, Evgenij Aleksandrovich, Sankt-Peterburg, 194355 (RU); BUSHUEV, Vladimir Aleksandrovich, Sankt-Peterburg, 194362 (RU); DUDAREV, Dmitrij Alekseevich, Sankt-Peterburg, 194295 (RU); ISAEV, Aleksandr Nikolaevich, Sertolovo, 188650 Leningradskaya oblast' (RU)
(74) Representative: Deambrogi, Edgardo
(86) International application number: PCT/RU2021/050239
(87) International publication number: WO 2022/098261

(57) **Abstract**

This technical solution relates to computer science.

The simulation complex for safe training of healthcare professionals during the pandemic comprises at least one XR station including a set of realistic VR scenarios to practice the personal safety skills by healthcare professionals during the pandemic, as well as the skills of diagnosis and treatment of patients with COVID-19; a computing center for centralized management, monitoring and accounting of the training sessions of healthcare professionals; at least one XR station, with the XR station comprising at least one system to ensure the user's full immersion in virtual reality, wherein the system to ensure the user's full immersion in virtual reality comprises at least the following: a wireless virtual reality headset and a set of wireless body motion capture sensors; a positional tracking system to determine the user's position and orientation in the virtual environment, wherein the tracking system contains at least 2 modules for determining the position and orientation of a user in a virtual environment, which are installed in the corners of the room in which safe training of healthcare professionals is carried out during the pandemic; an administration center comprising at least two computing devices, a display and a video receiver and transmitter.

## Description

### TECHNICAL FIELD

This technical solutions relates to computer science, particularly, to complexes for safe training of healthcare professionals during the pandemic.

### PRIOR ART

The prior art closest to the claimed solution is the solution RU 2715148 C1 dated February 25, 2020. This solution relates to computer science, namely, the virtual reality simulation equipment. The simulator comprises a PC with a machine-readable medium. It comprises the logical part of the simulator and the graphical three-dimensional graphical shell that are connected to the PC. It also comprises peripheral devices for navigation in the virtual environment. The logical part, being a software package, comprises the switching module and the evaluation module connected by means of a local software interface. Virtual Reality Commuting Simulator is configured so that the operator is connected by two-way communication with the instructor and the virtual reality headset. The virtual reality headset comprises a helmet, headphones, a microphone, a manipulator control system, a pose tracking system and a motion platform. The VR headset has a two-way connection to the software system. It comprises a scenario set module, a scenario editing module and a basic software core module. The engine module consists of a simulator module, a switching module, an evaluation module and a voice module.

However, it should be noted that in prior art, the information about the objective automated assessment of a trainee's actions by the administration center and the computing center for centralized management, monitoring and accounting of training sessions has not been disclosed.

The proposed solution is aimed at eliminating the drawbacks of prior art and is different from the known solutions by the fact that the suggested complex provides an objective automated assessment of the trainee's actions.

### SUMMARY OF THE INVENTION

The technical problem that is solved by the declared solution is the creation of a simulation complex for safe training of healthcare professionals during the pandemic. Additional variants of this invention implementation are presented in the dependent claims.

The technical result is provided by ensuring safe training of healthcare professionals during the pandemic. An additional technical result is the growth of the arsenal of technical means of the simulation complex.

The declared technical results are provided by creating the simulation complex for safe training of healthcare professionals during the pandemic, which comprises the following:
at least one XR station including a set of realistic VR scenarios to practice the personal safety skills by healthcare professionals during the pandemic, as well as the skills of diagnosis and treatment of patients with COVID-19;
a computing center for centralized management, monitoring and accounting of the training sessions of healthcare professionals; at least one XR station, with the XR station comprising at least one system to ensure the user's full immersion in virtual reality, wherein the system to ensure the user's full immersion in virtual reality comprises at least the following: a wireless virtual reality headset and a set of wireless body motion capture sensors; a positional tracking system to determine the user's position and orientation in the virtual environment, wherein the tracking system contains at least 2 modules for determining the position and orientation of a user in a virtual environment, which are installed in the corners of the room in which safe training of healthcare professionals is carried out during the pandemic; an administration center comprising at least two computing devices, a display and a video receiver and transmitter.

In a particular embodiment of the described simulation complex, an XR station further comprises sets of disposable sanitary pads to be used with the VR headset and body sensors.

### DESCRIPTION OF THE DRAWINGS

Implementation of the invention will be further described in accordance with the attached drawings, which are presented to clarify the essence of the invention and by no means limit the field of the invention. The following drawings are attached to the application:
Fig. 1 shows the user's motion capture sensors. Fig. 2 shows the computing center.

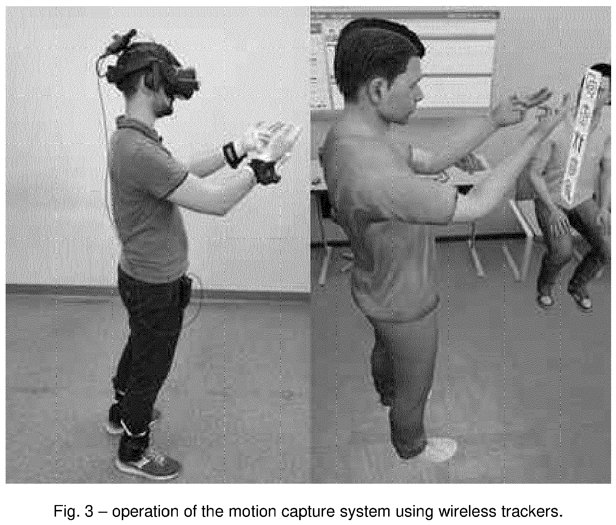

Fig. 3 shows the operation of the motion capture system using wireless trackers.

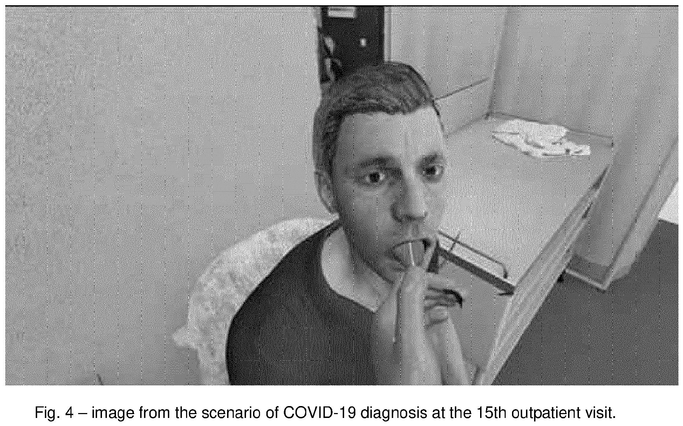

Fig. 4 shows an image from the scenario of COVID-19 diagnosis at the 15th outpatient visit.

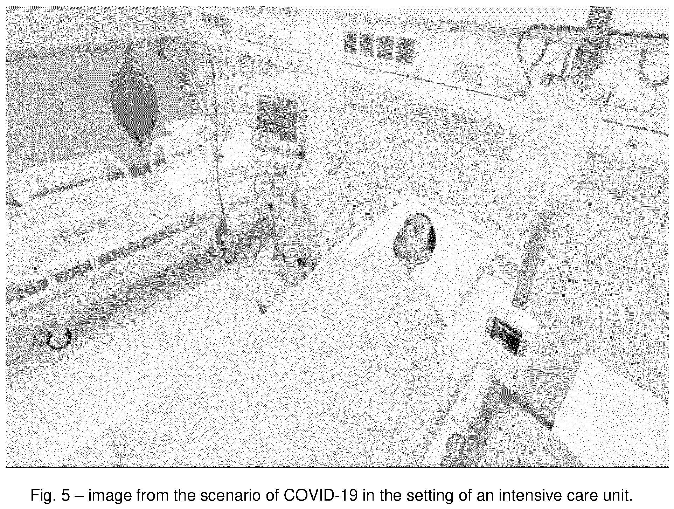

Fig. 5 shows an image from the scenario of COVID-19 in the setting of an intensive care unit.
Fig. 6 shows the computing device scheme.

### DETAILED DESCRIPTION OF THE INVENTION

Numerous implementation details intended to ensure clear understanding of this invention are listed in the detailed description of the invention implementation given next. However, it is obvious to a person skilled in the art how to use this invention as with the given implementation details as without them. In other cases, the well-known methods, procedures and components have not been described in details so as not to obscure unnecessarily the present invention.

Besides, it will be clear from the given explanation that the invention is not limited to the given implementation. Numerous possible modifications, changes, variations and replacements retaining the chief matter and form of this invention will be obvious to persons skilled in the art.

Computer technologies are actively used in the educational process almost all over the world. The creation of training computer simulators is one of the key areas in the computerization of learning. Full immersion in virtual reality and interaction with its objects is achieved only with the use of special devices. Such devices that provide full immersion in virtual reality and simulate human interaction with it using the senses are called virtual reality (VR) systems.

An important factor for increasing the level of immersion in the virtual environment is the possibility of interactive interaction with the elements of the environment and characters in virtual reality.

The proposed technical solution, using end-to-end digital technologies of virtual and augmented reality, provides continuous training of healthcare professionals in their work during the pandemic.

The simulation complex (XR center) for safe training of healthcare professionals during the pandemic transforms the procedures of simulation training of healthcare professionals.

The transformation mainly concerns ensuring safe social distancing for healthcare professionals that are being trained, minimization of the risks of transmission of all kinds of infections.

The end-to-end digital technologies of virtual and augmented reality:
- Provide the procedure of simulation training of healthcare professionals with high-quality immersion in the simulation environment;
- Train the users in the rules and methods of outpatient visits, biological safety and work in intensive care during the pandemic;
- Allow saving significant resources on equipping the simulation center;
- Distribute the flows of trainees to locations, providing the necessary social distancing and control of the assimilation of the material with the help of an objective automated assessment of the actions of the user. The user's actions are evaluated at the end of the training session. After each training sessions, the total score reflecting the correctness of the user's actions is automatically calculated;
- Reduce the number of physical objects that trainees come into contact with, minimizing the spread of infections;
- Exclude the presence of actors simulating the patient's behavior, which eliminates the additional risk of infection transmission.

The simulation complex allows significantly reducing the risk of the infection transmission during the training sessions of healthcare professionals (students, residents, physicians) and comprises the a distributed network of simulators with a unified system for managing, monitoring and recording training sessions.

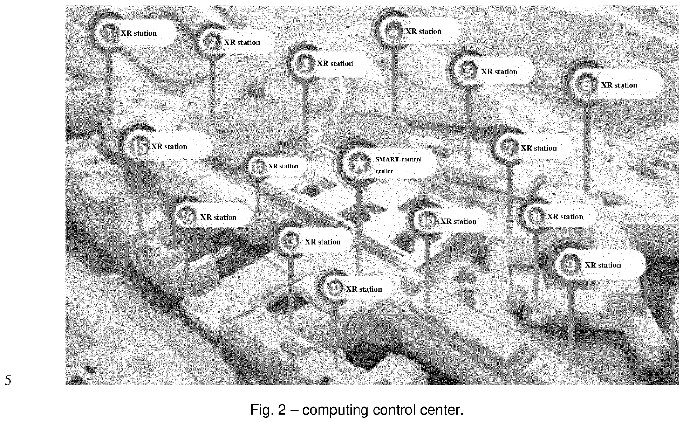

The computing center (Smart center) is shown in Fig. 2. The computing center is a unique system implementing the centralized monitoring, management and statistics of XR-station sessions distributed across various locations. It should also be taken into account that the number of XR stations may not be limited and all XR stations may be under the unified control of a computing center.

The functionality of the center comprises:
- Demonstration of XR stations on the map;
- Demonstration of the condition of the XR center at this moment;
- Access to the history of sessions and statistics;
- Access to surveillance video cameras in each XR station;
- Centralized access to trainees' accounts and to the results of their training sessions;
- Real-time monitoring of events in the virtual environment of XR stations;

XR-station session monitoring and control system provides for:
- Distribution of flows of trainees to reduce the risk of infection transmission during epidemics;
- Automated collection of statistical data linked to trainees' accounts;
- Portability, mobility and hygienic design of the equipment;
- Scenarios for training in diagnosis and personal safety when working with patients with suspected COVID-19.

XR-stations with cases (sets of scenarios) to combat COVID-19 are configured as stations for practicing the skills of personal safety by healthcare professionals during the pandemic. XR means "Extended Reality" or "Augmented Reality". A "station" is a widely accepted term used in medical simulation that can be interpreted either as an OSCE (Objective Structured Clinical Examination) station, or as a reference to the history of infectious diseases, where the term "station" was used to designated special anti-epidemic stations at hospitals or medical universities in the 19th century to combat the rabies epidemics.

XR station comprises:
- A system of full immersion in VR (wireless VR headset, a set of wireless body motion capture sensors).
- The positional tracking system (compact modules mounted in the corners of the room);
- The administration center comprising at least two computing devices, a display and a video receiver and transmitter;
- Sets of disposable sanitary pads to be used with the VR headset and body sensors.

An XR station comprises a set of highly realistic VR scenarios to practice the personal safety skills by healthcare professionals during the pandemic, as well as the skills of diagnosis and treatment of patients with COVID-19.

The set of scenarios includes:
- Practicing skills for ensuring physician's personal safety when working with COVID-19 infected patients, including working with personal protective equipment and disposal of waste products;
- Conducting medical office visits for patients with suspected COVID-19;
- Working with patients with COVID-19 of various severity in intensive care units using mechanical ventilators.

The administration center allows watching the images from surveillances video cameras mounted in each simulator, real-time monitoring of the learning process, examination of records of the training sessions and the user account database.

The comprehensive technology of full immersion into the VR is applied for the interaction with the VR environment. It implements a system for determining the position of the human body and its parts.

Owing to this process, the interactions of the trainee with the virtual reality occur naturally (without controllers).

The Atlas VR technology can be used as a comprehensive technology for full immersion in virtual reality, which allows all kinds of interactions with a virtual patient. Therefore, there is no need to use physical models (mannequins) or to include actors for physical examination (percussion, auscultation) and other tests, and to conduct surgical procedures on a virtual patient.

The trainee can easily move in the space, see him/herself, and interact with different interactive objects with one's own hands without any wires or controllers. Haptic gloves provide the possibility to feel the interaction with virtual objects and the patient.

The tracking technology system comprises the positional tracking system and a VR bodysuit.

The characteristics of the positional tracking system: coordinate output frequency: 90 Hz;
Coordinate determination sensitivity: 1 mm;
The number of users tracked at the same time: 4;
Possible scaling for the tracking cover of up to 100 m²;
The VR suit consists of a set of inertial sensors mounted on the user's body, and tracks angular coordinates of the user's body parts in real time.

The system for tracking the positions of users' hands and feet is implemented using a set of four sensors on each user's body.

Characteristics of the tracking system:
Orientation output frequency for each sensor: 100 Hz;
Angular resolution: 16 bit;
Range of measured angular velocities: +/- 2000 deg./sec; the range of measured accelerations: +/- 4g;
A runtime system that receives data into the tracking and suit system, calculates the final coordinates of key points, and transmits data to Unreal Engine 4;
Compatible with UE4 and Unity.

Gloves with haptic feedback provide vibration feedback during interactive actions. Additionally, there is an ergonomic button for performing the functions of capturing virtual 5 objects.

The user's body tracking system and the gloves allow the user to move naturally, interact with virtual objects and feel the tactile feedback.

At the same time, the technology implements algorithms for rapid automated calibration of the system for a specific user, which significantly improve the ergonomics and speed of connecting wearable sensors. The user stands up straight, hands at his sides. The administrator initiates the calibration process, and the system determines whether the sensors are correctly attached to the user, thereby simplifying and speeding up the process of connecting the user to the complex.

The completeness of immersion is additionally provided by libraries of models, animations and algorithms of game mechanics for the environment, interactive objects and virtual patients in the simulation.

The appearance of the patient's model corresponds to his/her state of health, which can be used to make a diagnosis. This is realized due to highly polygonal models, high-resolution textures, animation recorded by actors using Motion Capture technology, and voiceover of replicas, also performed by professional actors.

Using the full body tracking system, unlike classic VR joysticks, allows interacting naturally with patients and interactive objects. Such interaction does not require special training for the user, and also allows you to expand the range of interactions, including manipulations such as percussion, palpation, meaningful operation with a mechanical ventilator, etc.

At the same time, the proposed technical solution contains a system for integrating and increasing content, which allows quickly adding new clinical cases, which gives unlimited opportunities to expand the scenario library. This ensures a big difference in quality compared to competing solutions and allows setting a new technical standard in medical simulation.

The proposed solution allows studying at the highest level possible, carrying out the accreditation of specialists and acquiring the first professional skills without any risk to patients.

The use of the Customer Relationship Management (CRM) methodology provides training of psychomotor and sensorimotor skills, both technical and non-technical skills: communication, leadership, team resource management, working in a complex realistic environment of a hybrid operating room.

At the same time, the simulation complex provides better ergonomics due to the operation of the wireless simulator.

The operation of the simulator is implemented using two technologies:
- Wireless video transmission system for the virtual reality headset;
- A set of wireless trackers for tracking the position of the user's hands, feet and head.

The above-mentioned wireless technologies make the simulation complex compact, allow it to be deployed quickly in a limited space, and increase the throughput.

The following components can be used to ensure full immersion of the user in VR:
Wireless virtual reality headset (e.g., HTC Viva Pro) with a high-frequency video signal receiver;
A pair of infrared cameras for the arm tracking; basic Lighthouse tracking system stations; body sensors for the user's body motion capture;
A pair of wireless gloves (e.g., XR-Clinic Glove).

The simulation complex is additionally equipped with a router and an IP surveillance video camera.

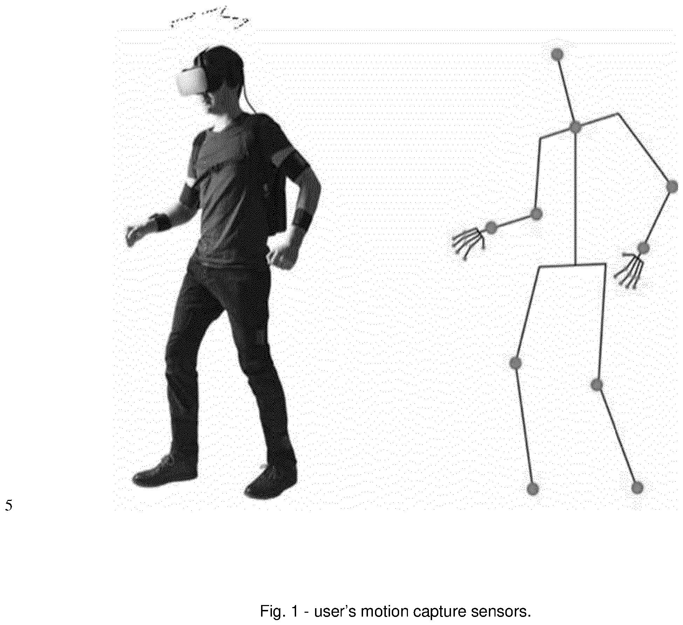

The main distinguishing features from known analogues are as follows:
- The use of the user's motion capture sensors (Fig. 1). The use of the algorithms for constructing the user's virtual avatar's skeleton providing for the capture of motions of the whole body including the arms, legs, and fingers.
- Wireless video transmission system for the virtual reality headset. That is why the user does not need to carry a backpack with a gaming computer or be tied with wires to a stationary system unit;
- A complex of four wireless sensors for capturing user movements and a universal calibration system that allow quickly attaching sensors without having to provide a specific orientation on the user's body;
- There is no need to carry VR controllers;
- There is no need to learn how to use the simulator due to natural interaction with the virtual environment (capturing virtual objects, moving around the room on your own feet, using tools as in real life).
- The use of the realistic dynamic virtual environment;
- Unique gloves designed specifically for the XR Clinic project, including a set of inertial sensors for tracking the orientation of the user's hands, a palm compression sensor for capturing virtual objects and a haptic feedback system. The gloves are connected to the system via a wireless radio channel;
- Interactive environment, including medical instruments, virtual patients, nurses;
- Automated system of objective evaluation of students' actions in accordance with the relevant recommendations of the Ministries of Health;
- Remote updates, including new scenarios, new locations, updates to the system of automated objective evaluation of trainees' actions, lists of medications, treatment protocols and results of examinations of virtual patients.

The means for developing the proposed technical solution were as follows:
Integrated development environment in C++ Visual Studio;
Integrated software development environment for microcontrollers in C CubelDE; Unreal Engine 4 game engine; 3DS Max 3D editor; SteamVR VR product development framework.

The methods and algorithms used in the development of the proposed solution were as follows:
Kalman filter;
Inverse kinematics algorithms;
Algorithms for automated calibration of sensors.

The XR station software comprises of two licenses:
- XR station administration license
- The XR station software comprises two licenses (XR station with cases to combat COVID-19) (10 cases)

The Smart Center software comprises a management system for 30 or more users.

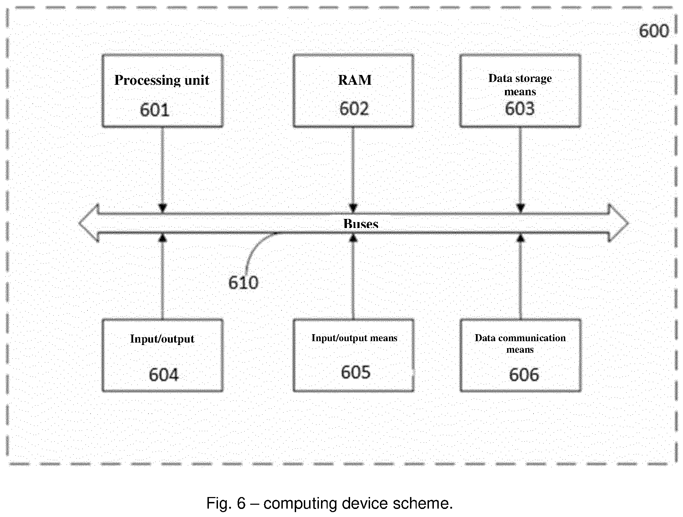

In Fig. 6, hereafter there will be presented the schematic diagram of the computer device (N00), processing the data, required for embodiment of the claimed solution.

In general, the device (600) comprises such components as: one or more processing units (601), at least one memory (602), data storage means (603), input/output interfaces (604), input/output means (605), networking means (606).

The device processing unit (601) executes main computing operations, required for functioning the device (600) or functionality of one or more of its components. The processing unit (601) runs the required machine-readable commands, contained in the random-access memory (602).

The memory (602), typically, is in the form of RAM and comprises the necessary program logic ensuring the required functional.

The data storage means (603) could be in the form of HDD, SSD, RAID, networked storage, flash-memory, optical drives (CD, DVD, MD, Blue-Ray disks), etc. The means (603) enables to store different information, e.g. the above-mentioned files with data sets obtained from different hosts, databases comprising records recorded in different periods for each user of time intervals, user identifiers, etc.

The interfaces (604) are the standard means for connection and operation with server side, e.g. USB, RS232, RJ45, LPT, COM, HDMI, PS/2, Lightning, FireWire, etc.

Selection of interfaces (604) depends on the specific device (600), which could be a personal computer, mainframe, server, server cluster, thin client, smartphone, laptop, etc.

A keyboard should be used as a means of In/Out data (605) in any embodiment of the system implementing the described method. There can be any known keyboard hardware: it could be as integral keyboard used in a laptop or netbook, as a separate device connected to a desk computer, server or other computer device. Provided that the connection could be either wired, when the keyboard connecting cable is connected to PS/2 or USB-port, located on the desk computer system unit, or wireless, when the keyboard exchanges data over the air, e.g. radio channel with a base station, which, in turn, is connected directly to the system unit, e.g. to one of USB-ports. Besides a keyboard the input/output means could also include: joystick, display (touch-screen display), projector, touch pad, mouse, trackball, light pen, loudspeakers, microphone, etc.

Networking means (606) are selected from a device providing network data receiving and transfer, e.g. Ethernet-card, WLAN/Wi-Fi module, Bluetooth module, BLE module, NFC module, IrDa, RFID module, GSM modem, etc. Making use of the means (605) provides an arrangement of data exchange through wire or wireless data communication channel, e.g. WAN, PAN, LAN, Intranet, Internet, WLAN, WMAN or GSM.

The components of the device (600) are interconnected by the common data bus (610).

The application materials have represented the preferred embodiment of the claimed technical solution, which shall not be used as limiting the other particular embodiments, which are not beyond the claimed scope of protection and are obvious to persons skilled in the art.

## Claims

1. The simulation complex for training of healthcare professionals during the pandemic comprising at least one XR station including a set of realistic VR scenarios to practice the personal safety skills by healthcare professionals during the pandemic, as well as the skills of diagnosis and treatment of patients with COVID-19; a computing center for centralized management, monitoring and accounting of the training sessions of healthcare professionals; at least one XR station, with the XR station comprising at least one system to ensure the user's full immersion in virtual reality, wherein the system to ensure the user's full immersion in virtual reality comprises at least the following: a wireless virtual reality headset and a set of wireless body motion capture sensors; a positional tracking system to determine the user's position and orientation in the virtual environment, wherein the tracking system contains at least 2 modules for determining the position and orientation of a user in a virtual environment, which are installed in the corners of the room in which safe training of healthcare professionals is carried out during the pandemic; an administration center comprising at least two computing devices, a display and a video receiver and transmitter.

2. The simulation complex according to claim 1, wherein an XR station additionally comprises sets of disposable sanitary pads to be used with the VR headset and body sensors.
